# EUROPEAN PATENT SPECIFICATION

(11) **EP 1 671 660 B1**
(45) Date of publication and mention of the grant of the patent: **29.07.2009**
(21) Application number: 05025345.9
(22) Date of filing: 21.11.2005
(51) Int. Cl.: A61L 24/04, A61L 15/58, A61K 9/70, C09J 7/02

(54) **Hot melt adhesives for medical application**
Schmelzklebemassen für medizinische Zwecke
Adhésifs thermofusibles pour applications médicales

(30) Priority: 24.11.2004 US 630915 P
(43) Date of publication of application: 21.06.2006
(73) Proprietor: Henkel AG & Co. KGaA, 40589 Düsseldorf (DE)
(72) Inventor: Paul, Charles W., Madison New Jersey 07940 (US); Palasz, Peter D., Taplow Maidenhead SL6 0LB (GB)
(74) Representative: Held, Stephan

(56) References cited:
- EP-A- 0 259 968
- EP-A- 0 342 808
- EP-A- 1 431 363
- WO-A-20/05080518
- WO-A-20/05080520
- WO-A-20/05080521
- US-A- 4 243 500
- US-A- 5 695 837

## Description

### FIELD OF THE INVENTION

The invention relates to an adhesive composition and end use applications thereof. In particular, UV-curable acrylic hot melt pressure-sensitive adhesives that are ideally suited for medical applications.

### BACKGROUND OF THE INVENTION

Pressure-sensitive adhesive (PSA) compositions are used for pressure-sensitive adhesive tapes, the adhesive tape comprising a backing and a PSA composition.

One field of where PSA compositions find wide spread use is the medical segment, e.g., various tapes, bandages and drug delivery devices. In many such applications, such as for example skin plasters, there is direct contact between the PSA composition and the patient's skin. Adhesives for application to the skin are permanently tacky at room temperature, hold the adhered article to the skin with gentle pressure, and should be easily removed without causing pain or depositing adhesive residue.

In medical applications, the requirements imposed on the PSA composition are especially stringent, since it is necessary to avoid skin irritation and allergic reaction. Moreover, such adhesives need to adhere well to human skin during perspiration, when the weather is hot, or in an environment of draining wounds.

There is an ongoing demand and continuing need in the art for PSAs useful in medical applications. The current invention addresses this need in the art.

### SUMMARY OF THE INVENTION

The invention relates to hot melt pressure sensitive adhesives compositions useful for medical applications. The adhesive is permanently adhered to at least one substrate of the article and removable or releasably attachable to skin.

One aspect of the invention provides pressure sensitive adhesives for use in medical applications. The adhesive may be used in the manufacture of articles such as such as plasters, bandages and tapes which are adhesively adhered to the skin.

The adhesive comprises a UV curable polymer and a tackifying resin, specifically a hydrocarbon resin. The UV curable polymer preferred for use is a UV curable acrylic polymer that comprises an acrylic copolymer covalently bound to a photoreactive group. A particularly preferred UV acrylic copolymer comprises 2-ethylhexyl acrylate and has bonded to it a pendant benzophenone group. Such UV acrylic copolymers are commercially available from BASF under the trade name acResin® A 204 UV.

In one embodiment the adhesive comprises a UV curable acrylic polymer and an aromatic hydrocarbon resin, most preferable an alpha methyl styrene resin having a softening point of less than about 110 °C. A particularly preferred tackifier for use with acResin® A 204 UV is Kristalex 3085 (Kristalex F85), an alpha-methyl styrene resin having a softening point of about 85°C which is commercially available from Eastman Chemical. Compositions comprising from about 75 wt % to about 90 wt % of polymer, and from about 10 wt % to about 25 % of tackifier are typical for use in the practice of the invention. Optionally, an antioxidant may, if desired, be added in amounts of up to about 3 wt %.

Another aspect of the invention provides articles such as such as plasters, bandages and tapes to be adhesively adhered to the skin. The articles comprise a backing substrate having coated to at least one surface thereof an adhesive comprising a UV curable polymer and a hydrocarbon tackifying resin. A UV curable polymer preferred for use is a UV curable acrylic polymer that comprises 2-ethylhexyl acrylate and a comonomer that contains a photoreactive group. Particularly preferred adhesives comprise a UV curable resin such as acResin® A 204 UV commercially available from BASF, and Kristalex 3085, an alpha methyl styrene resin commercially available from Eastman Chemicals.

Yet another aspect of the invention is directed to a method of administering a therapeutic agent to a patient comprising applying to a body surface of the patient a transdermal drug delivery system comprising a UV curable hot melt pressure sensitive adhesive and a physiologically active agent.

### DETAILED DESCRIPTION OF THE INVENTION

It has now been discovered that adhesive compositions prepared using particularly defined polymers and tackifiers can be used to manufacture PSA articles such as tapes and bandages used in medical applications.

The term "hot melt pressure-sensitive adhesive" or "hot melt pressure-sensitive adhesive composition" as used hereinafter means an adhesive or adhesive composition which, upon production of adhesive goods such as adhesive tapes and adhesive sheets by applying an adhesive or adhesive composition to a base material such as paper, cloth or plastic film, is capable of forming a layer of the pressure-sensitive adhesive or pressure-sensitive adhesive composition on the base material by applying it to the base material as a hot-melt.

The term "pressure-sensitive adhesive" is used herein to refer to a viscoelastic material which adheres instantaneously to most substrates with the application of slight pressure and remains permanently tacky.

The term "tackifier" as used herein means any composition which is useful to impart tack to the hot melt adhesive composition. ASTM D-1878-1T defines tack as "the property of a material which enables it to form a bond of measurable strength immediately on contact with another surface".

UV curable polymers are defined herein as polymers comprising a photoinitiator that is covalently bound to the polymer. Preferred are polymers comprising an acrylic polymer backbone molecule that is modified with polymerized photoreactive groups, e.g., a modified benzophenone group that is chemically bonded to the acrylic polymer chain. The polymer is crosslinked by chemical grafting caused by the excitation of the photoinitiator by UV irradiation.

UV curable polymers are commercially available from BASF under the trade name acResin® UV. These materials are solvent- and water-free acrylic raw material that can be used for the production of pressure sensitive tapes and labels. These polymers are nearly solid material at room temperature and have to be heated to a temperature of about 120-130°C to become fluid enough (viscosity ca. 40 Pa s) for the coating process on paper or plastic carriers. At this temperature, they can be applied to the backing substrate or carrier with conventional hot melt coating systems. Thus they are processed as hot melts. After being coated on the carrier, the polymer film is crosslinked by UV-irradiation to produce the adhesive properties required.

One of these polymers, specifically an unmodified (i.e., not tackified) acResin® A 258 UV, has been recommended for use in medical applications. The acResin® A 258 UV product comprises butyl acrylate with no 2-ethylhexy acrylate component.

Applicants have now discovered that the use of UV curable polymers is surprising useful in medical applications when it is used with a tackifying resin. Particularly preferred are UV curable polymers comprising 2-ethylhexyl acrylate, such as acResin® A 204 UV. Particularly preferred is acResin® A 204 UV polymer. This polymer has not heretofore been used in medical applications. Applicants have discovered that the use of this polymer in combination with hydrocarbon tackifiers are especially useful in medical applications. Since the photoinitiator is one of the monomers that forms a part of the polymer chain, migration of the photoinitiator is avoided as is the toxicity associated with their presence.

Levels of UV curable polymers is generally from about 70 wt % to about 90 wt %, more preferably from about 75 wt % to about 85 wt %.

Tackifiers that are useful in the practice of the invention are hydrocarbon resin, primarily aromatic hydrocarbon (% of aromatic protons via 1H NMR of >15%, preferably >30, most preferably >50). Tackifying resins that can be used in the practice of the invention include Kristalex F110, 115, 120 and 3085(F85)available from Eastman. Preferred are tackifying resins that have a softening point of less than about 110°C, more preferably below about 90°C. Particularly preferred for use in the practice of the invention is Kristalex 3085(F85).

Levels of tackifiers is generally from about 1 wt % to about 30 wt %, more preferable from about 10 to about 25 wt % and more preferably from about 15 wt % to about 22.5 wt %.

The compositions of the invention may include other additives known to those skilled in the art. These additives may include, but are not limited to, pigments, fillers, fluorescent additives, flow and leveling additives, wetting agents, surfactants, antifoaming agents, rheology modifiers, stabilizers, and antioxidants. Preferred additives are those which do not have appreciable absorption in the wavelengths of interest.

Antioxidants are typically added to protect the ingredients against degradation during preparation and use of the adhesive compositions and to ensure long-term thermal stability, however without interfering with the irradiation curing of the polymer.

Combinations of antioxidants are often more effective due to the different mechanisms of degradation to which various polymers are subject. Certain hindered phenols, organo-metallic compounds, aromatic amines, aromatic phosphites, and sulphur compounds are useful for this purpose. Examples of effective types of these materials include phenolic antioxidants, thio compounds, and tris(nonylated phenyl) phosphites.

In general up to 3 % by weight of one or more antioxidants is included in the adhesive compositions. Usually, 0 to about 3 wt %, preferably from about 0.1% to about 3% by, more preferably from about 0.4% by weight to about 1.5% by weight.

Representative antioxidants that may be used in the practice of the invention include: 1,3,5-trimethyl 2,4,6-tris (3,5-di-tert-butyl-4-hydroxybenzyl) benzene; pentaerythrityl tetrakis-3(3,5-di-tert-butyl-4-hydroxyphenyl)-propionate; 4,4'-methylenebis (2,6-tertbutylphenol); 4,4'-thiobis (6-tert-butyl-o-cresol); 2,6-di-tert-butylphenol; 2-t-butyl-6-(3-t-butyl-2-hydroxy-5-methylbenzyl)-4-methylphenyl acrylate, 6-(4-hydroxyphenoxy)-2,4-bis(n-octylthio)-1,2,5-triazine; di-n-octadecyl 3,5-di-tert-butyl-4-hydroxybenzyl phosphonate; 2-(n-octylthio)ethyl 3,5-di-tert-butyl-4-hydroxybenzoate; and sorbitol hexa[3-(3,5-di-tert-butyl-4-hydroxyphenyl)-propionate]. Such compounds are commercially available Ciba.

The UV curable polymer and tackifier (as well as other desired components such as antioxidant) are blended together at a temperature of from about 130°C, but not more than 150°C, until a clear mixture is formed. Entrapped air may be removed by application of a vacuum.

Following coating of the composition onto a carrier such as paper or foil, it is subjected to UV irradiation. Under the action of UV light, the photoreactive groups in the UV curable polymer crosslink the polymer backbone.

Conventional medium pressure mercury-vapor lamps which emit UV wavelengths can be used in the practice of the invention to cure the adhesives of the invention.

The resultant pressure sensitive adhesive articles are useful as ostomy seals, adhesive tapes and bandages, wound drainage adhesive seals, wound dressings, as adherents for other products and the like that adhere to human skin and remain adherent even in a moist environment.

The adhesive of the invention is also well-suited for use in transdermal applications. The pressure sensitive adhesive of the invention may be incorporated into a transdermal drug delivery device designed to deliver a therapeutically effective amount of a product to the skin of a patient, e.g., to cure a skin irritation or to deliver a therapeutically effective amount of drug across the skin of a patient. The term transdermal refers to the use of the skin as a portal for the administration of drugs by topical application. The topically applied drug passes into and/or through the skin. Thus "transdermal" is used broadly to refer to the topical administration of a drug which acts locally, i.e., at the surface or within the skin, such as, for example, a blemish patch used to treat acne, and to the topical application of a drug which acts systemically by diffusing through the skin and entering the blood stream.

The adhesive of the invention is contemplated for use in the manufacture of liquid reservoir patches and matrix patches.

Transdermal drug delivery devices of the invention comprise a carrier (such as liquid, gel, or solid matrix, or a pressure sensitive adhesive) into which the drug to be delivered is incorporated, a distal backing layer and a proximal release layer. When the patient peels the release liner from the adhesive and applies the patch, the drug partitions into the stratum corneum (outer skin layer) and permeates through the epidermis and dermis.

Backings which can be used in the practice of this invention include, with or without modification, metal foils, metalized polyfoils, composite foils or films containing poytetrafluoroethylene (TEFLON®)-type materials or equivalents thereof, polyether block amide copolymers, polyurethanes, polyvinylidene chloride, nylon, silicone elastomers, rubber-based poylisobutylene styrene, styrenebutadiene and styrene-isoprene copolymers, polyethylene, polyester, and other such materials used in the art of transdermal drug delivery. Particularly preferred are thermoplastic polymers such as polyolefins, for example polyethylene and polypropylene, and polyesters such as polyethyleneterephthalate.

The term "drug" is to be construed herein in its broadest sense to mean any agent which is intended to produce some therapeutic benefit. The agent may or may not be pharmaceutically active, but will be "bioactive" in the sense that it has an effect on the human body. The agent may be used to treat or alter a condition, which may or may not be a pathological, i.e., a disease state. "Drug", "bioactive agent," "preparation," "medicament," "therapeutic agent," "physiological agent" and "pharmaceutical agent" are used interchangeably herein and include substances for use in the diagnosis, cure, mitigation, arrest, treatment or prevention of a condition or disease state or to affect the structure or function of the body. Skin-wellness agents that function to e.g., soften and moisturize are included in this term. The term "treatment" is used broadly to encompass prevention, alteration, cure and control of the condition.

The drug is present in a drug delivery device of the invention in a therapeutically effective amount, i.e., an amount effective to bring about a desired therapeutic result in the treatment of a condition to which the preparation of this invention is to be applied. Effective amount of a drug means a nontoxic but sufficient amount of a drug to provide the selected effect over a specific period of time. The amount that constitutes a therapeutically effective amount varies according to the particular drug incorporated in the device, the condition being treated, any drugs being co-administered with the selected drug, desired duration of treatment, the surface area of the skin over which the device is to be placed, and other components of the drug delivery device. Such an amount is readily determinable by the skilled practitioner.

The drug delivery system of the invention, in addition to the drug, may advantageously also contain an effective amount of a penetration enhancer. An effective amount of a penetration enhancer means an amount that provides a selected increase in membrane permeability, rate of administration and amount of drug.

The device of the invention is placed on the skin and allowed to remain for a time sufficient to achieve or maintain the intended therapeutic effect. The time that constitutes a sufficient time can be selected by those skilled in the art with consideration of the flux rate of the device of the invention and of the condition being treated.

The transdermal delivery devices of the invention can be made in the form of an article such as a tape, a patch, a sheet, a dressing or any other form known to those skilled in the art. The dosage system may be produced in any desirable unit form. A circular form is convenient as it contains no corners which might be easily detached from the skin. In addition to having various shapes, the dosage units produced may come in various sizes.

Depending on the design of the patch and the condition to be treated, the patch will remain on the skin for up to an hour or more, up to about one week. In a preferred embodiment, the patch is designed to remain on the skin at the application site for about 24 hours, and to be changed daily. Preferably, the patch will be placed on the skin at a site different from the location of the previously used patches.

The invention will be described further in the following examples, which are included for purposes of illustration and are not intended, in any way, to be limiting of the scope of the invention.

### EXAMPLES

Test Methods and UV Cure

### UV Cure

Adhesive films were cured using medium pressure mercury arc lamps. The dose was measured with an EIT Power Puck. UVC is the region between 200 and 280 nm. Benzophenone photoinitiator groups have a peak absorbance in this region.

### Shear

The adhesive was cast as a 2 mil film on a 1.5 mil PET backing film. This was then cut into strips, each having a 1 square inch bonded area and bonded to a polished stainless steel plate. Shear was then measured using 1 Kg and 2 Kg weights.

### Peel

The Adhesive was cast as a 2 mil film on a 1.5 mil PET backing film. Peel Adhesion was measured as the force required to remove a pressure sensitive tape from a standard PSTC stainless steel panel at a specified angle and speed. Required force being expressed in pounds per inch width of tape. Equipment used included a standard PSTC 4½ lb rubber-covered roller, and a standard Instron Tensile Testing Machine.

The following procedure was followed: a stainless steel panel was cleaned as per standard PSTC method. The coating to be tested was conditioned for 24 hours at 72°F(±2°F) and 50% (±5%) Relative Humidity (RH). Test strips having the dimension 1" x 6" were cut. The rubber roller was washed with hexane to remove any dirt or oil. About 1" of release liner was removed from the test specimen and applied to the panel and reinforced with 1" wide masking tape. The remainder of release liner was removed from the test specimen and the rubber roller passed over it in each lengthwise direction at a speed of 12" per minute using only the pressure of the weight of the roller (any samples showing the presence of air bubbles were discarded). The test specimen, attached to the panel, was aged for 20 minutes at 72°F(±2°F) and 50% (±5%) RH. The Instron was set with a crosshead speed of 12" per minute. The free end of the tape was doubled back at an angle of 180°, clamped to the upper jaw of the Instron and reinforced with masking tape. The end of the panel was clamped to the lower jaw of the Instron. The test strip was then peeled from the panel and the peel force recorded in pounds per inch width of tape.

### Loop Tack

A 2 mil adhesive film was coated onto a 1.5 mil PET backing film. The film was then coated with release liner, and cut into 1" x 5" strips. A test sample was then inserted into a TMI tester. The tester automatically recorded the tack value in oz/in². SAFT (Shear Adhesion Failure Temperature)

Shear samples were prepared as described above. These samples were allowed to equilibrate in an oven for 15 minutes, in most cases starting at 180°F - 200°F, and then a 1 kilogram weight was hung. Subsequently the temperature of the oven was increased 10°F every 15 minutes until failure was noted or 300°F was reached.

The two materials (acResin® A 204 UV and tackifier) were blended together at a temperature of about 130°C. Entrapped air was removed by application of a vacuum.

All coatings were 2 mils on a 1.5 mil PET backing. All bonds were made automatically using a Chemsultants automated roller (4.5 1b).

Sample 1 contained 80 wt % of acResin® A 204 UV and 20 wt % of Kristalex F85 (hydrocarbon resin).

Sample 2 contained 90 wt % of acResin® A 204 UV and 10 wt % of Kristalex F85.

Comparative Sample 3 contained 80 wt % of acResin® A 204 UV and 20 wt % of Foral 85 (rosin ester tackifier available from Eastman).

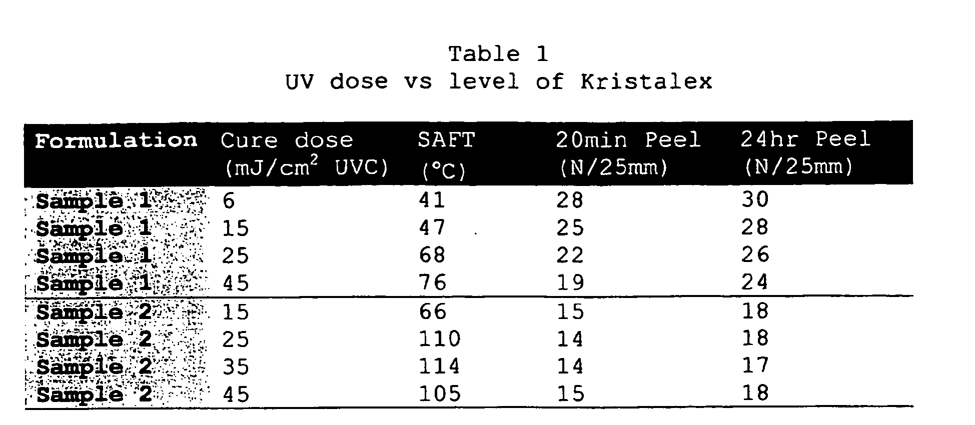

The data set forth in Table 1 shows that high level of Kristalex gives higher peel adhesion values and lower SAFT. UV dose controls the SAFT and Peel values. Low level of Kristalex improves SAFT, but peel adhesion values do not change.

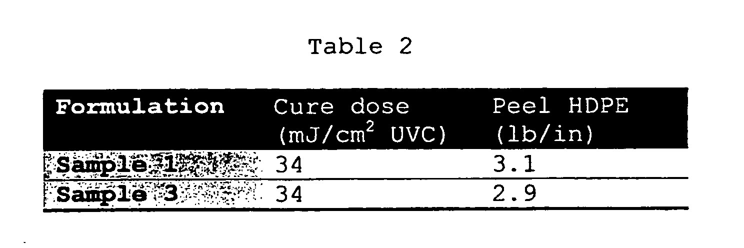

Skin is a hydrophobic surface and therefore peel on high density polyethylene (HDPE) is often used as an indicator of peel on skin. Peel values above 2 1b/in are considered high compared to conventional non-UV acrylic tapes. The data in Table 2 as well as tests on human skin show that both adhesives bond well to skin.

While the data in Table 2 shows that rosin esters may also be useful in medical applications, these tackifiers are derived from natural sources and may cause skin irritations due to trace impurities. As such, the use of hydrocarbon resins is preferred. The results set forth in Table 2 show the hydrocarbon resin-containing adhesive (Sample 1) has higher peel on HDPE than the rosin ester - containing adhesive (Sample 3).

## Claims

1. A pressure sensitive adhesive for use in medical applications comprising a UV curable polymer, a hydrocarbon resin tackifier and optionally other additives, wherein the UV curable polymer comprises a covalently bonded photoinitiator.

2. The adhesive of claim 1, wherein the level of the UV curable polymer is from about 70 to 90 wt.-% and the level of the hydrocarbon resin tackifier is from about 1 to 30 wt.-%.

3. The adhesive of claim 2, wherein the level of the UV curable polymer is from about 75 to 85 wt.-% and/or the level of the hydrocarbon resin tackifier is from about 10 to 25 wt.-%.

4. The adhesive of one of claims 1 to 3 wherein the UV curable polymer is a UV curable acrylic polymer.

5. The adhesive of claim 4, wherein the UV curable acrylic polymer comprises 2-ethylhexyl acrylate.

6. The adhesive of claim 5, wherein the UV curable acrylic polymer comprises 2-ethylhexyl acrylate and a comonomer, that contains a photoreactive group.

7. The adhesive of claim 6, wherein the photoreactive group is a pendant benzophenone group.

8. The adhesive of one of claims 1 to 7, wherein the tackifier resin is an aromatic hydrocarbon resin.

9. The adhesive of claims 8, wherein the aromatic hydrocarbon resin is an alpha methyl styrene resin having a softening point of less than about 110°C.

10. An article to be adhesively adhered to the skin, which article comprises a backing substrate having coated to at least one surface thereof an adhesive according to at least one of the preceding claims, the polymer backbone of the adhesive being crosslinked by UV irradiation.

11. The article of claim 10 which is a tape, a plaster, a bandage, or a drug delivery device.

12. The article of claim 10, which is a transdermal drug delivery system.

13. The article of claim 10, which is a medical tape.

## Patentansprüche

1. Haftklebstoff zur Verwendung in medizinischen Anwendungen, umfassend ein UV-härtbares Polymer, einen Kohlenwasserstoffharz-Klebrigmacher und gegebenenfalls andere Zusatzstoffe, wobei das UV-härtbare Polymer einen kovalent gebundenen Photoinitiator umfasst.

2. Klebstoff nach Anspruch 1, wobei der Gehalt des UV-härtbaren Polymers ungefähr 70 bis 90 Gew.-% und der Gehalt des Kohlenwasserstoffharz-Klebrigmachers ungefähr 1 bis 30 Gew.-% betragen.

3. Klebstoff nach Anspruch 2, wobei der Gehalt des UV-härtbaren Polymers ungefähr 75 bis 85 Gew.-% und/oder der Gehalt des Kohlenwasserstoffharz-Klebrigmachers ungefähr 10 bis 25 Gew.-% betragen.

4. Klebstoff nach irgendeinem der Ansprüche 1 bis 3, wobei das UV-härtbare Polymer ein UV-härtbares Acrylpolymer ist.

5. Klebstoff nach Anspruch 4, wobei das UV-härtbare Acrylpolymer 2-Ethylhexylacrylat umfasst.

6. Klebstoff nach Anspruch 5, wobei das UV-härtbare Acrylpolymer 2-Ethylhexylacrylat und ein Comonomer umfasst, das eine photoreaktive Gruppe enthält.

7. Klebstoff nach Anspruch 6, wobei die photoreaktive Gruppe eine Benzophenon-Seitengruppe ist.

8. Klebstoff nach irgendeinem der Ansprüche 1 bis 7, wobei das Klebrigmacherharz ein aromatisches Kohlenwasserstoffharz ist.

9. Klebstoff nach Anspruch 8, wobei das aromatische Kohlenwasserstoffharz ein Alphamethylstyrolharz mit einem Erweichungspunkt unter ungefähr 110°C ist.

10. Artikel, haftend an der Haut anzubringen, wobei der Artikel ein Trägersubstrat umfasst, das an mindestens einer Oberfläche davon mit einem Klebstoff nach mindestens einem der vorangehenden Ansprüche beschichtet ist, wobei die Polymerhauptkette des Klebstoffs durch UV-Bestrahlung vernetzt ist.

11. Artikel nach Anspruch 10, welcher ein Klebeband, ein Pflaster, ein Verband oder eine Arzneimittelabgabevorrichtung ist.

12. Artikel nach Anspruch 10, welcher ein transdermales Arzneimittelabgabesystem ist.

13. Artikel nach Anspruch 10, welcher ein medizinisches Klebeband ist.

## Revendications

1. Adhésif autocollant à utiliser dans des applications médicales, comprenant un polymère durcissable par exposition au rayonnement ultraviolet, un agent poisseux à base de résine d'hydrocarbure et, de manière facultative, d'autres additifs, dans lequel le polymère durcissable par exposition au rayonnement ultraviolet comprend un photoinitiateur lié de manière covalente.

2. Adhésif selon la revendication 1, dans lequel le taux du polymère durcissable par exposition au rayonnement ultraviolet s'élève d'environ 70 à 90 % en poids et le taux de l'agent poisseux à base de résine d'hydrocarbure s'élève d'environ 1 à 30 % en poids.

3. Adhésif selon la revendication 2, dans lequel le taux du polymère durcissable par exposition au rayonnement ultraviolet s'élève d'environ 75 à 85 % en poids et/ou le taux de l'agent poisseux à base de résine d'hydrocarbure s'élève d'environ 10 à 25 % en poids.

4. Adhésif selon l'une quelconque des revendications 1 à 3, dans lequel le polymère durcissable par exposition au rayonnement ultraviolet est un polymère acrylique durcissable par exposition au rayonnement ultraviolet.

5. Adhésif selon la revendication 4, dans lequel le polymère acrylique durcissable par exposition au rayonnement ultraviolet comprend de l'acrylate de 2-éthylhexyle.

6. Adhésif selon la revendication 5, dans lequel le polymère acrylique durcissable par exposition au rayonnement ultraviolet comprend de l'acrylate de 2-éthylhexyle et un comonomère qui contient un groupe photoréactif.

7. Adhésif selon la revendication 6, dans lequel le groupe photoréactif est un groupe latéral de benzophénone.

8. Adhésif selon l'une quelconque des revendications 1 à 7, dans lequel la résine qui rend poisseux est une résine d'hydrocarbure aromatique.

9. Adhésif selon la revendication 8, dans lequel la résine d'hydrocarbure aromatique est une résine d'α-méthylstyrène possédant un point de ramollissement inférieur à environ 110 °C.

10. Article que l'on doit faire adhérer à la peau à l'aide d'un adhésif, ledit article comprenant un substrat de support sur au moins une des surfaces duquel est enduit un adhésif selon au moins une des revendications précédentes, le squelette polymère de l'adhésif étant réticulé par exposition au rayonnement ultraviolet.

11. Article selon la revendication 10, à savoir une bande, un emplâtre, un pansement ou un dispositif de distribution de médicament.

12. Article selon la revendication 10, à savoir un système transdermique de distribution de médicament.

13. Article selon la revendication 10, à savoir une bande médicale.
